# FASCICULE DE BREVET EUROPEEN

(11) **EP 3 793 471 B1**
(45) Date de publication et mention de la délivrance du brevet: **27.07.2022**
(21) Numéro de dépôt: 19733850.2
(22) Date de dépôt: 20.05.2019
(51) Int. Cl.: A61B 50/20, A61C 8/00, A61B 17/86, A61B 17/88, A61B 50/30, A61B 50/00

(54) **DISPOSITIF POUR LE MAINTIEN ET LA LIBERATION D'UN OBJET, ENSEMBLE ET PROCEDE DE LIBERATION CORRESPONDANTS**
VORRICHTUNG ZUM HALTEN UND FREIGEBEN EINES GEGENSTANDES, ENTSPRECHENDE ANORDNUNG UND FREIGABEVERFAHREN
DEVICE FOR HOLDING AND RELEASING AN OBJECT, CORRESPONDING ASSEMBLY AND RELEASE METHOD

(30) Priorité: 18.05.2018 FR 1854199
(43) Date de publication de la demande: 24.03.2021
(73) Titulaire: Selenium Medical, 17000 La Rochelle (FR)
(72) Inventeur: RICHART, Olivier, 17580 Le Bois Plage en Re (FR)
(74) Mandataire: Ipsilon
(86) Numéro de dépôt international: PCT/FR2019/051142
(87) Numéro de publication internationale: WO 2019/220067

(56) Documents cités:
- WO-A1-2015/179931
- WO-A1-2015/179931
- DE-U1-202007 004 638
- DE-U1-202014 100 438
- US-A1- 2013 103 150

## Description

### DOMAINE DE L'INVENTION

La présente invention concerne de manière générale un dispositif permettant de maintenir un objet, tel qu'un implant médical, et de libérer ledit objet par rapport à ce dispositif. L'invention concerne aussi un ensemble correspondant et un procédé de libération d'objet correspondant.

### ART ANTERIEUR

On connaît de l'état de la technique des dispositifs de maintien d'objet, encore appelés préhenseurs, qui permettent de coupler un implant médical audit dispositif. L'opérateur peut saisir le dispositif et désactiver le couplage de l'objet au dispositif pour saisir l'implant.

Les documents WO 2015/179931, DE 20 2014 100438 U1, DE 20 2007 004638 et US 2013/103150 A1 décrivent de tels préhenseurs.

Il est souhaitable de pouvoir proposer d'autres dispositifs de maintien d'objet pour lesquels l'objet peut être maintenu de manière fiable, tout en permettant de le libérer aisément du dispositif, et en limitant le risque de libération intempestive.

### RESUME DE L'INVENTION

A cet effet, il est proposé un dispositif pour le maintien et la libération d'un objet selon la revendication 1.

La force de rappel exercée par au moins l'un des systèmes de crochet sur l'objet, permet de maintenir l'objet en contact non seulement avec ledit système de crochet mais aussi avec l'autre système de crochet.

Ainsi l'objet reste maintenu de manière fiable dans le dispositif par les systèmes de crochet, notamment en l'absence de sollicitation extérieure venant déplacer l'un des ou les systèmes de crochet en position de libération, ou en l'absence de sollicitation volontaire sur l'objet, telle qu'une sollicitation à pivotement de la part de l'opérateur pour faire pivoter l'objet dans le sens d'une libération par rapport aux systèmes de crochet.

Le dispositif peut être logé dans un emballage ou double-emballages tel que décrit dans la demande internationale WO2014188142 dont le contenu est incorporé par référence à la présente demande ou la demande internationale WO2018078242 dont le contenu est incorporé par référence à la présente demande.

Le dispositif peut aussi comporter une ou plusieurs des caractéristiques suivantes prises dans toute combinaison techniquement admissible.

Selon une caractéristique particulière, à l'état couplé dudit objet auxdits systèmes de crochet, chacun desdits systèmes de crochet étant soumis à une force de rappel, lesdits systèmes de crochet sont configurés de telle sorte que la force de rappel auquel est soumis l'un desdits systèmes de crochet, tend à le déplacer en sens opposé au sens de la force de rappel auquel est soumis l'autre système de crochet, l'objet étant maintenu couplé auxdits systèmes de crochet sous l'effet des forces de rappel exercées en sens opposé par lesdits systèmes de crochet sur l'objet.

Selon un aspect particulier, chaque système de crochet tend à ramener l'objet en direction opposée à l'autre système de crochet.

En particulier, chaque système de crochet tend à ramener l'objet du côté du bras dudit système de crochet.

Selon une caractéristique particulière, au moins l'un, de préférence chacun, des systèmes de crochet est configuré pour pouvoir être déplacé par rapport à l'autre système de crochet dans une position, dite position de libération, permettant de libérer l'objet par rapport aux systèmes de crochet.

En particulier, en position de libération, la ou les forces de rappel précédemment exercées sur le ou les systèmes de crochet sont réduites ou annulées, ce qui facilite la libération de l'objet vis-à-vis des systèmes de crochet.

Selon une caractéristique particulière, au moins l'un, de préférence chacun, des systèmes de crochet comprend un bras qui est muni d'au moins un crochet, et ledit ou chaque bras est déplaçable, de préférence par flexion ou déformation élastique, pour permettre au système de crochet correspondant, d'être déplacé par rapport à l'autre système de crochet.

Selon un mode de réalisation, le déplacement des systèmes de crochet en position de libération s'effectue dans le sens d'un rapprochement des bras des systèmes de crochet l'un par rapport à l'autre.

Selon une caractéristique particulière, le dispositif comprend un corps auquel est couplé chaque système de crochet.

Chaque système de crochet présente au moins un crochet présentant un passage à travers lequel au moins une partie dudit objet est apte à s'étendre. En particulier, ledit passage dudit au moins un crochet de chaque système de crochet présente un axe de passage le long duquel au moins une partie dudit objet est apte à s'étendre.

Selon une caractéristique particulière, les crochets sont, à l'état couplé de l'objet auxdits systèmes de crochets, répartis à différentes positions le long dudit axe de passage.

Lesdits crochets sont ainsi disposés de manière à crocheter (ou entourer partiellement) différentes parties ou portion de l'objet.

Autrement dit chaque système de crochet comprend au moins un crochet qui, à l'état couplé de l'objet auxdits systèmes de crochets, est écarté dudit au moins un crochet de l'autre système de crochet le long d'un axe parallèle à l'axe du logement du corps destiné à recevoir une partie de l'objet.

Le passage délimité par chaque crochet et à travers lequel s'étend l'objet, à l'état couplé aux systèmes de crochet, présente un axe de passage qui est sensiblement parallèle à l'axe du logement ménagé dans le corps et destiné à recevoir au moins une partie de l'objet.

Avantageusement, dans le cas d'un mode de réalisation prévoyant un déplacement du bras du système d'accrochage par flexion (déformation élastique) autour d'un axe correspondant, ledit axe du passage du crochet est transversal, de préférence orthogonal, à l'axe de flexion du ou de chaque système d'accrochage.

Selon une caractéristique particulière, ledit ou chaque système de crochet est déplaçable par flexion autour d'un axe orthogonal audit axe de passage. En particulier, ledit axe autour duquel est déplaçable ledit ou chaque système de crochet est aussi orthogonal au plan moyen du corps ou du capot.

Ledit au moins un crochet de chaque système de crochet présente une zone d'ouverture de crochet configurée de manière à permettre une libération dudit objet à travers ladite zone d'ouverture de crochet, par pivotement dudit objet autour d'un axe orthogonal à l'axe de passage. Selon un aspect particulier, l'axe de pivotement pour la libération de l'objet est aussi parallèle au plan moyen du corps ou du capot.

Selon une caractéristique particulière, l'un des systèmes de crochet comprend un crochet supplémentaire, à travers lequel au moins une partie dudit objet est apte à s'étendre, ledit au moins un crochet de l'autre système de crochet étant apte à s'étendre, à l'état couplé de l'objet aux systèmes de crochet, entre les deux crochets dudit système de crochet.

Selon un aspect particulier, l'axe de passage du crochet supplémentaire extérieur est coaxial avec l'autre crochet du système de crochet correspondant.

A l'état couplé de l'objet aux systèmes de crochet, lesdits crochets sont répartis à différentes positions le long de l'axe de passage des crochets.

Selon une caractéristique particulière, au moins l'un des crochets présente une portion d'extrémité qui s'étend de manière à, lorsque l'objet est couplé aux systèmes de crochet en étant soumis à la force de rappel du ou de chacun des systèmes des crochets, s'opposer à une libération de l'objet par déplacement à translation dans une direction contenue dans un plan orthogonal à l'axe selon lequel s'étend l'objet à l'état maintenu de l'objet par lesdits systèmes de crochet.

Selon une caractéristique particulière, la portion d'extrémité de ce crochet comprend une rampe qui permet la libération de l'objet par pivotement de l'objet autour d'un axe orthogonal à l'axe selon lequel est maintenu ledit objet par les systèmes de crochet.

Selon une caractéristique particulière, les systèmes de crochet sont configurés pour permettre la libération de l'objet par pivotement de l'objet autour d'un axe orthogonal à l'axe selon lequel est maintenu ledit objet par les systèmes de crochet.

Selon une caractéristique particulière, chaque crochet présentant une ouverture, dite ouverture d'insertion/libération, définie entre la base du crochet et l'extrémité libre de sa partie recourbée, l'ouverture d'au moins l'un, d'une partie, ou de chacun, des crochets présente sensiblement la même orientation que l'ouverture d'au moins l'un, d'une partie, ou de chacun des autres crochets des systèmes de crochet.

Autrement dit, chaque crochet présente une partie d'accrochage qui entoure partiellement l'objet, à l'état couplé dudit objet aux systèmes de crochet. Chaque crochet présente aussi une partie ouverte. Cette partie ouverte correspond à l'espace laissé libre entre l'extrémité libre de la partie d'accrochage et le début de la partie d'accrochage qui fait la liaison entre le bras du système d'accrochage et la partie d'accrochage correspondante.

Cette partie ouverte du crochet (c'est-à-dire l'ouverture d'insertion/libération) forme une ouverture radiale par rapport à l'axe du passage défini par le crochet et le long duquel l'objet s'étend. L'objet peut être sorti du crochet par cette ouverture radiale, en particulier lors d'un pivotement de cet objet autour d'un axe transversal à l'axe de passage du crochet.

Selon un aspect particulier, l'ouverture d'insertion/libération de chaque crochet est ouverte selon une direction transversale à l'axe de passage du crochet et à l'axe de pivotement de l'implant qui permet sa libération par passage par ladite ouverture d'insertion/libération.

La répartition des crochets à différentes positions axiales par référence à l'axe de passage, à l'état couplé de l'objet aux systèmes de crochet, fait que leurs ouvertures d'insertion/libération sont aussi réparties à différentes positions axiales.

Selon une caractéristique particulière, le support est phosphorescent ou luminescent pour être visible dans l'obscurité, en particulier dans un environnement de bloc opératoire.

Selon une caractéristique particulière, le dispositif comprend également un capot monté coulissant entre une position, dite position de masquage, qui masque au moins partiellement les systèmes de crochet, et une autre position, dite position d'accès aux systèmes de crochet, qui permet à l'opérateur de déplacer les systèmes de crochet.

Selon une caractéristique particulière, le capot est configuré de telle sorte que, en position de masquage, la longueur de l'objet, c'est-à-dire au moins ses extrémités opposées, reste visible.

L'invention concerne également un ensemble comprenant un dispositif tel que proposé ci-dessus, et un objet maintenu par lesdits systèmes de crochet.

L'ensemble peut aussi comporter une ou plusieurs des caractéristiques suivantes prises dans toute combinaison techniquement admissible.

Selon une caractéristique particulière, ledit objet, de préférence un implant médical, comprend un corps de vis allongé et une tête de vis, les systèmes de crochet venant en prise avec le corps de vis allongé.

Selon un aspect particulier, la tête de vis présente un diamètre supérieur au corps de vis, ce qui permet à ladite vis de venir en butée contre un crochet, en particulier le crochet situé le plus vers l'extérieur du dispositif.

Selon une caractéristique particulière, l'extrémité du corps de vis est contenue dans un logement ménagé dans un corps du dispositif.

Avantageusement, l'axe de ce logement est sensiblement parallèle ou coaxial à l'axe de passage des crochets à l'état couplé de la vis aux systèmes de crochet.

L'invention concerne également un procédé selon la revendication 15.

Il est aussi proposé un dispositif pour le maintien et la libération d'un objet, tel qu'un implant médical, caractérisé en ce que le dispositif comprend deux systèmes de crochet avec lesquels ledit objet est apte à être couplé pour son maintien, lesdits systèmes de crochet étant configurés de telle sorte que, à l'état couplé dudit objet auxdits systèmes de crochet, chaque système de crochet est soumis à une force de rappel qui tend à le déplacer en sens opposé au sens de la force de rappel auquel est soumis l'autre système de crochet, l'objet étant maintenu couplé auxdits systèmes de crochet sous l'effet des forces de rappel exercées en sens opposé par lesdits systèmes de crochet sur l'objet.

### BREVE DESCRIPTION DES DESSINS

D'autres caractéristiques et avantages de l'invention ressortiront encore de la description qui suit, laquelle est purement illustrative et non limitative et doit être lue en regard des dessins annexés, sur lesquels :

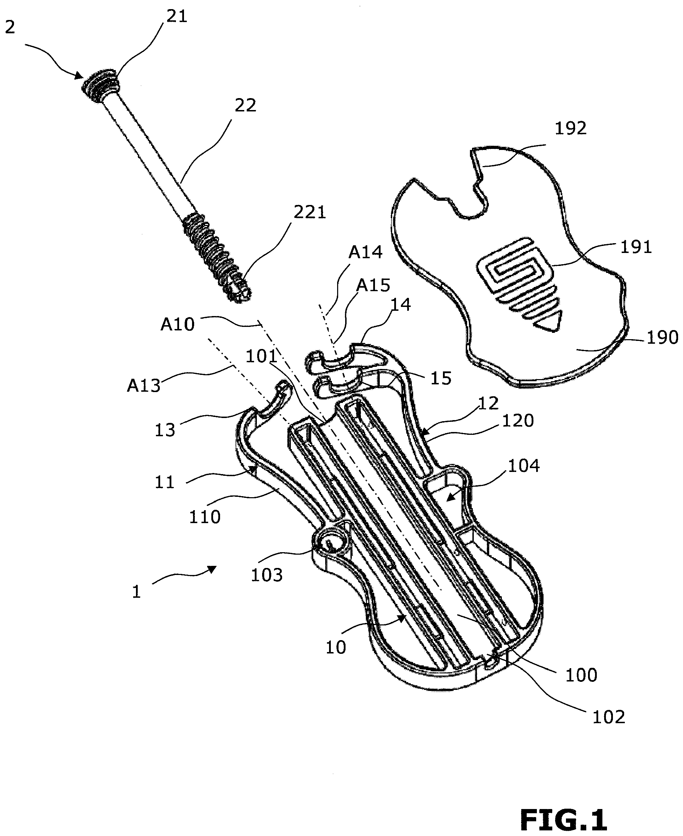

- la figure 1 est une vue éclatée d'un ensemble comprenant un dispositif de maintien et un implant médical conformément à un mode de réalisation de l'invention ;
- la figure 2 est une vue conformément à un mode de réalisation d'un dispositif de maintien et d'un implant maintenu dans le dispositif par des systèmes de crochet, le dispositif comprenant un capot en position fermée/masquage ;
- la figure 3 est une vue correspondant au dispositif de la figure 2 en une position ouverte du capot ;
- la figure 4 est une vue d'un dispositif et d'un implant conformément à un mode de réalisation montrant un opérateur en train de pincer avec deux doigts les systèmes de crochet pour libérer l'implant ;
- la figure 5 et une vue conformément à un mode de réalisation d'un dispositif de maintien et d'un implant médical, un outil étant amené pour être couplé à l'implant et, de préférence après avoir pincé les systèmes de crochets, extraire l'implant du dispositif par exemple par déplacement axial de l'implant ;
- la figure 6 et une vue conformément à un mode de réalisation d'un dispositif de maintien et d'un implant médical, en position ouverte du capot, un outil étant amené pour libérer l'implant du dispositif par pivotement ;
- la figure 7 est une vue d'un ensemble comprenant un dispositif et un implant conformément à un mode de réalisation de l'invention, l'implant étant en prise avec les crochet soumis à des forces de rappel ;
- la figure 7A est une vue de détail de la figure 7 ;
- la figure 8 est une vue du dispositif et de l'implant conformément à un mode de réalisation de l'invention, en cours de libération de l'implant par pivotement autour d'un axe orthogonal à l'axe de l'implant et parallèle au plan moyen du capot ;
- la figure 8A est une vue de détail de la figure 8 ;
- la figure 9 est une vue en perspective d'un ensemble comprenant un implant et un dispositif conformément à un mode de réalisation de l'invention, à l'état retiré de l'implant par rapport au dispositif ;
- la figure 9A est une vue de détail de la figure 9.

### DESCRIPTION DETAILLEE

Le concept de l'invention est décrit plus complètement ci-après avec référence aux dessins joints, sur lesquels des modes de réalisation du concept de l'invention sont montrés. Sur les dessins, la taille et les tailles relatives des éléments peuvent être exagérées à des fins de clarté. Des numéros similaires font référence à des éléments similaires sur tous les dessins. Cependant, ce concept de l'invention peut être mis en œuvre sous de nombreuses formes différentes et ne devrait pas être interprété comme étant limité aux modes de réalisation exposés ici. Au lieu de cela, ces modes de réalisation sont proposés de sorte que cette description soit complète, et communiquent l'étendue du concept de l'invention aux hommes du métier. Les modes de réalisation qui suivent sont examinés, par souci de simplification, en relation avec la terminologie et la structure d'un dispositif permettant de maintenir et de libérer un objet, tel qu'un implant médical.

En référence aux figures, on a représenté un dispositif 1 pour le maintien et la libération d'un objet 2.

Dans l'exemple illustré aux figures, ledit objet 2 est un implant médical. L'implant peut ou non comprendre une partie mobile par rapport à une autre partie de l'implant. L'implant médical peut comprendre un corps 22 de vis et une tête 21 de vis. La tête de vis peut être fixe par rapport au corps de vis 22, ou mobile, par exemple avec une liaison de type rotule. Ainsi, dans la suite de la description, le dispositif est décrit en lien avec l'implant 2, mais la description s'applique bien entendu aussi à un autre objet apte à être maintenu par le dispositif et libéré.

Le dispositif peut encore être appelé préhenseur. Un tel dispositif peut être utilisé par un opérateur pour manipuler un objet par l'intermédiaire du dispositif sans toucher l'objet avec ses doigts.

Comme détaillé ci-après, le dispositif dans lequel est tenu l'implant 2 permet aussi de libérer l'implant 2 qui peut alors être extrait du dispositif par exemple à l'aide d'un outil 9 adapté, par exemple un outil de type tournevis, présentant un embout apte à coopérer avec la tête 21 de l'implant, pour coupler l'implant 2 à l'outil 9.

### • Systèmes de crochet

Dans l'exemple illustré aux figures, le dispositif comprend deux systèmes 11, 12 de crochet qui permettent de maintenir ensemble l'implant 2, avant libération.

L'implant 2 est apte à être couplé aux systèmes de crochet 11, 12 pour son maintien dans le dispositif.

Chaque système 11, 12 de crochet peut comprendre un bras 110, 120 muni d'au moins un crochet 13, 14 à travers lequel au moins une partie 22 de l'implant 2 est apte à s'étendre.

Au moins l'un, de préférence chacun, des bras 110, 120 est déplaçable pour permettre au système 11, 12 de crochet correspondant, d'être déplacé par rapport à l'autre système 12, 11 de crochet. Selon un aspect particulier, l'un ou chacun des bras, et donc l'un ou chacun des systèmes de crochet, sont déplaçables par flexion ou déformation élastique. Autrement dit, l'opérateur peut pincer le ou les bras pour le ou les faire pivoter ou fléchir l'un vers l'autre.

Le dispositif permet de libérer l'implant en actionnant les systèmes de crochets d'une seule main avec deux doigts. Les systèmes de crochet peuvent être configurés de manière à permettre un tel actionnement des systèmes de crochet aussi bien pour un droitier que pour un gaucher. On peut prévoir que l'effort à appliquer à un bras ou système de crochet pour le déplacer soit sensiblement de même valeur que celui pour déplacer l'autre bras ou système de crochet dans le sens d'un rapprochement des bras ou systèmes de crochet.

La description qui suit est réalisée pour un mode de réalisation selon lequel les deux bras ou systèmes de crochet sont déplaçables, de préférence par flexion ou déformation élastique, mais la description s'applique aussi au cas où un seul bras ou système de crochet est déplaçable, de préférence par flexion ou déformation élastique.

Le déplacement des bras ou systèmes de crochet permet d'amener les crochets dans une position permettant que l'implant soit couplé aux crochets pour son maintien dans le dispositif par coopération des deux systèmes de crochet ou dans une position de libération dudit implant.

Selon un aspect particulier, chaque système 11, 12 de crochet présente au moins un crochet 13, 14 présentant un passage à travers lequel au moins une partie 22 dudit objet 2 est apte à s'étendre.

Le système de crochet 12 peut comprendre un crochet 15 supplémentaire, à travers lequel au moins une partie 22 dudit implant 2 est apte à s'étendre. Le crochet 15 est écarté du crochet 14 selon l'axe A14, A15 de passage du crochet 14 ou 15. L'implant 2 est apte à s'étendre au moins en partie à travers ces deux crochets 14, 15.

Lorsque l'implant 2 est en prise avec les systèmes 11, 12 de crochet, le crochet 13 du système 11 de crochet s'étend entre les deux crochets 14, 15 dudit système 12 de crochet.

Une telle conception des systèmes 11, 12 de crochet selon laquelle un crochet d'un système de crochet est imbriqué entre deux autres crochets de l'autre système de crochet, permet de maintenir l'implant 2 de manière fiable. En particulier, le risque de libération intempestive de l'objet du fait d'un pivotement qui résulterait des forces de rappel opposés appliquées à l'objet, est réduit.

Pour autant, comme expliqué ci-dessus, il est prévu qu'un opérateur puisse libérer de manière volontaire l'implant 2 par application d'un effort de pivotement prédéfini autour d'un axe qui est sensiblement orthogonal à l'axe A2 de l'implant 2 et qui est contenu dans un plan parallèle au plan moyen dans lequel s'étendent les bras 110, 120 (ou encore le plan moyen du corps ou du capot).

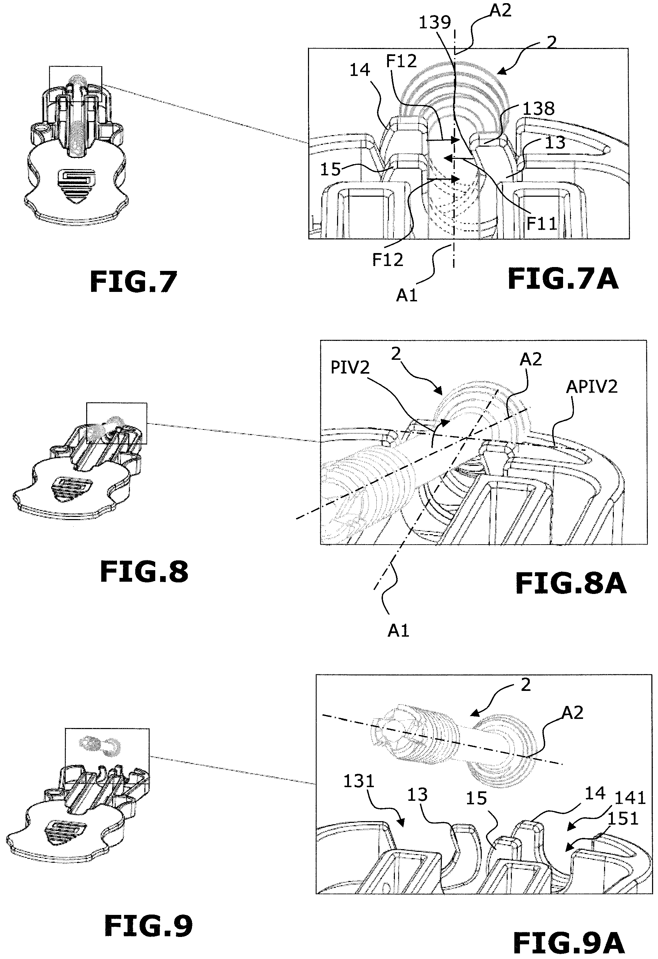

Comme illustré à la figure 9A, chaque crochet 13, 14, 15 présente une ouverture 131, 141, 151, dite ouverture d'insertion/libération, définie entre la base du crochet et l'extrémité libre de sa partie recourbée. Avantageusement, chaque ouverture 131, 141, 151 de crochet présente sensiblement la même orientation que l'ouverture du ou de chaque autre crochet des systèmes 11, 12 de crochet.

L'ouverture 131, 141, 151 de crochet peut en particulier être ménagée dans une zone opposée à la zone du crochet sur laquelle repose l'implant 2 lorsque ledit implant 2 est couplé au système 11, 12 de crochet et à l'état positionné du dispositif sur une surface horizontale. Les ouvertures des crochets sont alors situées au-dessus des zones de repos de l'implant dans les crochets.

Autrement dit, lorsque l'opérateur tient le dispositif face à lui, c'est-à-dire avec le capot 190 (présenté ci-après) orienté vers lui, les ouvertures d'insertion/libération de chaque crochet sont situées sensiblement du côté de l'opérateur.

Selon un mode de réalisation, l'un 13 des crochets présente une portion d'extrémité 138 dont la forme permet de s'opposer à une tentative de libération de l'objet 2 par déplacement à translation dans une direction contenue dans un plan orthogonal à l'axe A1 selon lequel s'étend l'implant 2 à l'état maintenu de l'implant 2 par lesdits systèmes 11, 12 de crochet. En position de maintien de l'implant 2, l'axe A1 est sensiblement parallèle à l'axe de passage A13, A14, A15 défini par les crochets.

Selon un aspect particulier, la portion d'extrémité 138 de ce crochet 13 comprend une rampe 139 qui permet de favoriser la libération de l'implant 2 par pivotement PIV2 de l'objet 2 autour d'un axe APIV2 orthogonal à l'axe A1 selon lequel est maintenu ledit implant 2 par les systèmes 11, 12 de crochet. L'axe APIV2 (virtuel) peut être situé sensiblement au niveau du crochet 14.

On peut aussi prévoir que l'opérateur puisse faire sortir l'implant du dispositif en orientant le dispositif avec l'implant vers le bas de sorte que lorsque l'opérateur déplace l'un où chacun des systèmes de crochet pour libérer l'implant qui glisse alors par gravité par rapport à exemple sur une zone de réception située en-dessous du dispositif. On peut aussi prévoir que, sans inclinaison particulière, l'opérateur libère l'implant par déplacement de l'un où chacun des systèmes de crochet et extrait l'implant manuellement ou à l'aide d'un outil (qui peut être couplé à l'implant avant libération).

La tête 21 de vis s'étend en saille de l'ensemble formé par les crochets 13, 14, 15 qui entourent partiellement le corps 22 de vis allongé.

### • Corps du dispositif

Comme illustré aux figures, le dispositif comprend un corps 10. Le corps 10 peut présenter un axe longitudinal A10, qui peut être sensiblement parallèle à l'axe A1 présenté ci-après. Chaque bras 110, 120 présente une extrémité qui est fixée au corps 10 du dispositif. Le corps 10 peut s'étendre au moins en partie entre les deux bras 110, 120. Chaque bras 110, 120 présente une autre extrémité munie d'un crochet 13 ou de plusieurs crochets 14, 15. Comme rappelé ci-dessus, on peut prévoir que chaque bras puisse être déplacé, par flexion ou déformation élastique, vers l'autre bras.

Le corps peut présenter un logement 100 permettant de contenir au moins une partie 22 de l'implant 2. Ce logement 100 peut présenter la forme d'une gorge allongée dans l'axe du corps 10. La gorge peut comprendre une extrémité 101 pour permettre à l'implant 2 de s'étendre à travers cette extrémité ouverte 101 dans le logement 100.

Le logement 100 peut présenter une autre extrémité 102 au moins partiellement fermée de manière à empêcher une sortie de l'implant 2 par cette autre extrémité 102.

Lorsque l'opérateur active les systèmes 11, 12 de crochet en position de libération par exemple en les pinçant de manière à les rapprocher l'un de l'autre de sorte que les crochets 13, 14,15 n'empêchent plus la sortie de l'implant 2, les zones de fond des crochets 13, 14, 15 peuvent encore servir de surface de support de l'implant 2. Si le dispositif est incliné avec les crochets 13, 14,15 orientés vers le haut alors que les systèmes 11, 12 de crochet sont en position de libération, l'implant pourrait alors glisser par gravité. Dans ce cas, l'implant 2 peut être reçu dans le logement 100 du corps 10, ce qui évite le risque de chute au sol de l'implant 2.

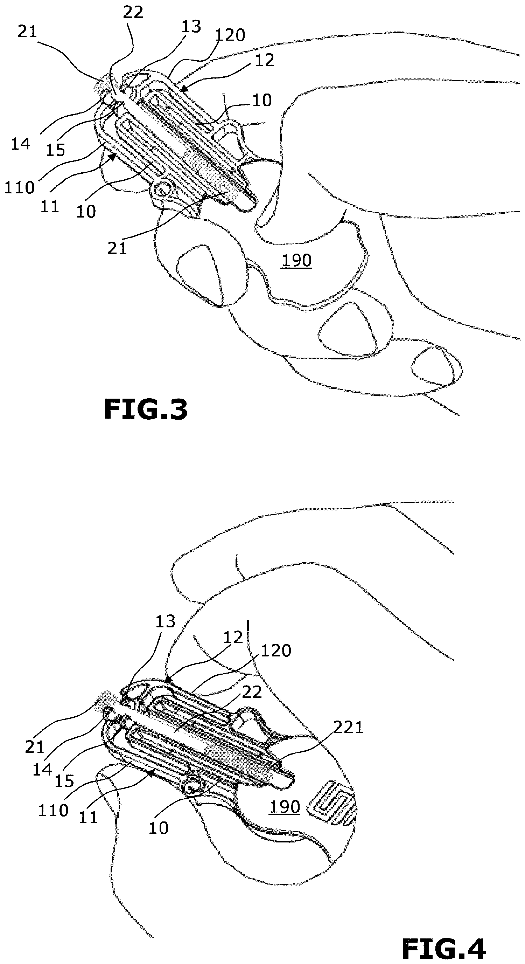

Selon un aspect particulier, le corps 10 peut aussi comprendre au moins un autre logement par exemple pour loger de petits implants (par exemple de type vis de couverture pour implant dentaire). Ainsi, dans l'exemple illustré aux figures, en particulier à la figure 4, le corps comprend deux autres logements 103, 104, un 103 en forme de rond et l'autre 104 en forme générale de goutte.

Dans l'exemple illustré aux figures, l'extrémité 221 libre du corps de vis opposée à la tête 21 de vis s'étend du côté du corps 10 principal du dispositif, de préférence dans le logement 100 ménagé dans le corps principal pour recevoir ladite extrémité.

Le logement 100 permet ainsi de guider une éventuelle chute de l'implant 2 dans le corps 10 principal du dispositif. On peut aussi prévoir que la tête de vis soit dimensionnée pour pouvoir venir en butée contre un crochet.

Le fait que l'extrémité du corps de vis s'étend dans le logement 100 permet aussi de limiter le risque de dégradation de l'environnement du dispositif. En particulier le risque de perforation d'un sachet, potentiellement sous vide, qui contiendrait le dispositif est réduit puisque l'extrémité du corps de vis est protégée par le corps principal du dispositif.

Comme expliqué ci-dessus, le corps 10 du dispositif peut permettre également de loger un ou plusieurs autres objets. Le dispositif peut être configuré pour permettre d'autoriser ou non l'accès au(x) logement(s) 103, 104 en fonction de la position du capot 190 (présenté ci-après). Comme rappelé ci-dessus, on peut ainsi prévoir que le corps 10 comprenne un ou plusieurs logements d'une ou plusieurs pièces et que ce ou ces logements soient refermés par le capot en position de masquage du capot, et éventuellement dans une position intermédiaire du capot dans laquelle ce ou ces logements restent masqués alors que l'implant et/ou les systèmes de crochet est(sont) suffisamment découvert(s) par rapport au capot pour permettre la libération de l'implant. L'opérateur peut alors continuer à déplacer le capot jusqu'à découvrir le ou les logements 103, 104.

### • Capot

Le dispositif peut comprendre un capot 190 monté coulissant entre une position, dite position de masquage, qui masque au moins partiellement les systèmes 11, 12 de crochet, et une autre position, dite position d'accès aux systèmes de crochet, qui permet à l'opérateur de déplacer les systèmes 11, 12 de crochet. On peut prévoir que, en position d'accès aux systèmes de crochet, le ou les logements 103, 104 restent masqués et que l'opérateur doive poursuivre le déplacement du capot pour accéder à ce ou ces logements 103, 104.

A cet effet, le dispositif peut comprendre un système de coulissement. Le système de coulissement peut comprendre des ergots ou nervures ménagées sur le capot qui viennent se mettre dans des glissières, formées par exemple par des rainures dans le corps 10, de préférence de part et d'autre du logement 100. Comme évoqué ci-dessus, le système de coulissement permet d'obtenir, outre la position fermée de masquage, et la position d'accès aux systèmes de crochet, une position d'ouverture supplémentaire en continuant le coulissement (descente) du capot pour accéder à un ou des logements supplémentaires tels que les logements 103 et 104.

Il est à noter que le capot 190 en position de masquage (position fermée) empêche une libération intempestive de l'implant. Le capot permet d'empêcher ou gêner l'accès de l'opérateur aux systèmes de crochet afin de prévenir une libération intempestive de l'objet par déplacement non intentionnel des systèmes de crochet ou par basculement par pivotement non intentionnel autour de l'axe APIV2 (virtuel) illustré à la 8A.

En position normale de saisie du dispositif par l'opérateur, c'est-à-dire en vue de dessus du dispositif et en position de masquage du capot, au moins une partie des systèmes de crochet est masquée par le capot 190.

Le capot 190 peut être configuré de telle sorte que, en position de masquage, la longueur de l'implant, c'est-à-dire au moins sa tête et son extrémité filetée, reste visible. La longueur de l'implant peut ainsi rester visible au moins pour l'opérateur qui regarde le dispositif de dessus en configuration normale de saisie du dispositif.

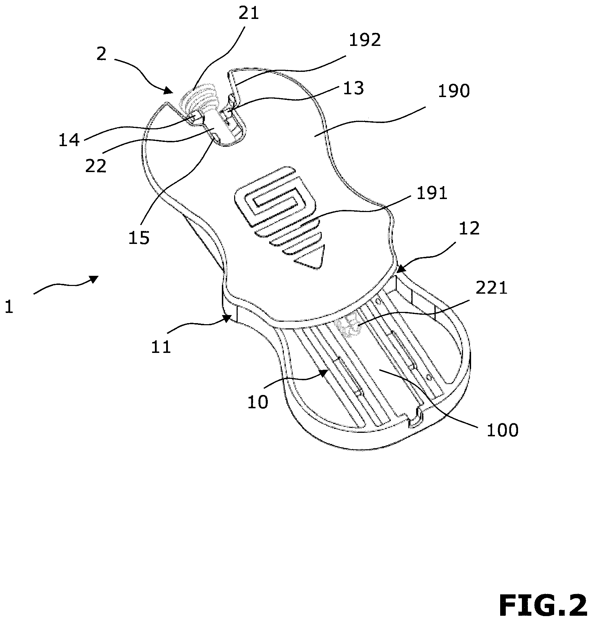

En particulier, le capot présente une encoche ou échancrure 192 qui permet de voir une partie d'extrémité d'implant, par exemple la tête 21 de vis et le début du corps de vis 22 dans l'exemple illustré à la figure 2.

Le capot 190 est muni d'une zone 191 de positionnement d'un doigt, encore appelée empreinte, par exemple comprenant des irrégularités de surface en creux ou en relief telles que des nervures ou stries, facilitant, l'entrainement en déplacement à coulissement du capot par l'opérateur.

### • Forces de rappel

Comme illustré à la figure 7A, lorsque l'implant 2 est maintenu dans le dispositif en étant couplé aux systèmes 11, 12 de crochet, le ou chaque crochet d'un système 11, 12 de crochet est soumis à une force F11, F12 de rappel qui tend à le déplacer en sens opposé au sens de la force F12, F11 de rappel auquel est soumis le ou chaque crochet de l'autre système 12, 11 de crochet. On peut aussi prévoir qu'un seul des systèmes de crochet soit déplaçable et soit soumis à une force de rappel lorsque l'implant est en prise avec les systèmes de crochets. Les caractéristiques présentées dans la description, notamment ci-après, s'appliquent aussi à un tel mode de réalisation.

L'implant 2 est maintenu couplé auxdits systèmes 11, 12 de crochet sous l'effet des forces F11, F12 de rappel exercées en sens opposé par les crochets desdits systèmes 11, 12 de crochet sur l'objet 2.

Les forces de rappel opposées auxquelles est soumis l'implant 2 du fait de son couplage avec les crochets 13, 14, 15 des systèmes 11, 12 de crochet, permet de maintenir l'objet à l'intérieur des crochets 13, 14, 15 des systèmes 11, 12 de crochet.

Lorsque l'implant 2 est en prise avec les crochets 13, 14, 15 des systèmes de crochet, les axes A13, A14, A15 de passage des crochets, sont sensiblement parallèles et éventuellement coaxiaux. En particulier les axes A13, A14, A15 sont alors sensiblement parallèles à l'axe A1 selon lequel l'implant 2 est maintenu dans le dispositif. Cet axe A1 est sensiblement confondu à l'axe A2 de l'implant 2 dans l'exemple illustré aux figures.

Selon un aspect particulier, les forces F11, F12 de rappel sont des forces de rappel élastique.

Ainsi lorsque la partie 22 de l'implant 2 s'étend au travers des crochets 13, 14, 15, le crochet 13 et les crochets 14,15 ont tendance à tirer sur la partie 22 de l'implant en sens opposé du fait des forces F11, F12 de rappel auxquelles lesdits crochets 13, et 14, 15 sont soumis. L'implant est ainsi maintenu en position à travers les crochets par coopération des crochets avec l'implant.

La forme supérieure d'un crochet, par exemple du crochet 14 peut avoir une forme adaptée, de préférence de section non circulaire, par exemple avec des pans coupés, pour coopérer avec une forme de l'implant de manière à limiter ou empêcher la rotation de l'implant lui-même autour de l'axe de son corps ou d'une partie de l'implant, dans le cas d'un implant présentant une partie mobile. On peut ainsi prévoir qu'avec une tête de vis montée avec liaison rotule sur son corps de vis, la tête de vis qui fait saillie des crochets soit bloquée en rotation par rapport à son corps grâce à une forme particulière du crochet 14 avec laquelle peut coopérer la tête de vis.

Le ou les crochets de chacun des systèmes 11, 12 de crochet est configuré pour pouvoir être déplacé par rapport à le ou chaque crochet de l'autre système de crochet 12, 11 dans une position, dite position de libération, permettant de libérer l'objet par rapport aux systèmes 11, 12 de crochet.

Les crochets 13, 14, 15 sont configurés pour être déplacés dans une position l'un par rapport à l'autre permettant de libérer l'implant par rapport aux crochets.

La position de libération peut être obtenue par déplacement d'au moins l'un, de préférence de chacun, des systèmes 11, 12 de crochet dans un sens opposé à la force de rappel auquel est soumis ledit système 11, 12 de crochet.

L'opérateur peut en effet appuyer avec ses doigts sur les systèmes 11, 12 de crochet de manière à les rapprocher en agissant à l'encontre des forces de rappel des systèmes de crochet.

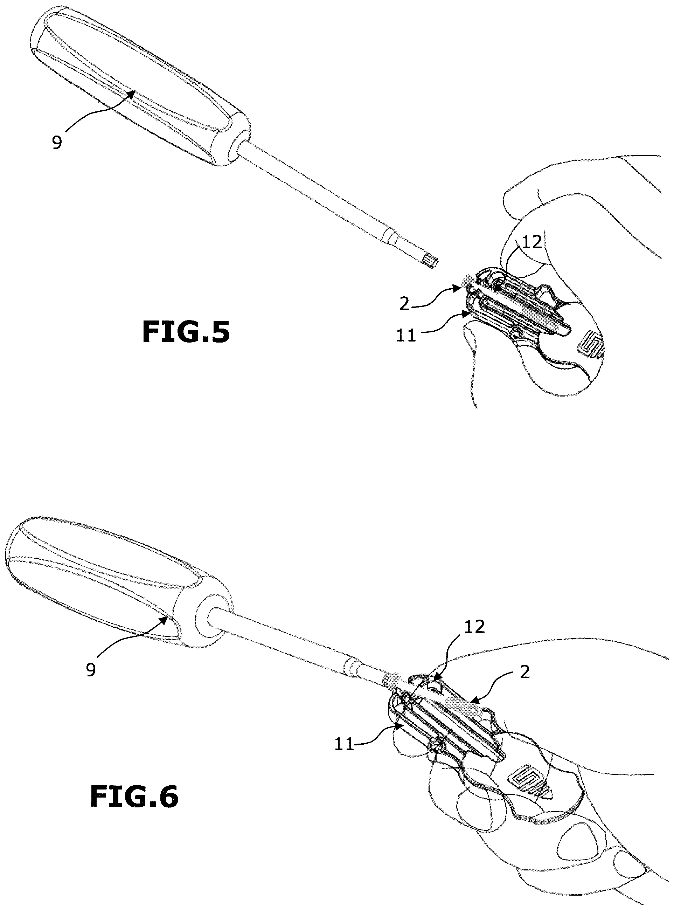

En particulier, l'opérateur peut rapprocher l'un de l'autre les bras 110, 120 des systèmes 11, 12 de crochet en pinçant lesdits bras entre deux doigts comme illustré aux figures 4 et 5.

Dans le cas où l'un des systèmes de crochet n'est pas déplaçable, ledit système de crochet étant alors dit fixe, on peut prévoir que l'opérateur libère l'implant en déplaçant l'autre système de crochet dans le sens d'un rapprochement du système de crochet fixe.

On peut aussi prévoir comme illustré à la figure 8, que l'implant puisse être enlevé par pivotement de l'implant, par exemple autour d'un point ou axe virtuel situé au niveau du crochet 14, sans qu'il soit nécessaire de presser ou d'exercer une force inverse à la force de rappel des systèmes 11, 12 de crochet.

Un dispositif tel que décrit ci-dessus permet de procéder à la libération de l'implant 2 de la manière suivante. L'opérateur déplace le capot 190 pour permettre l'accès aux systèmes 11, 12 de crochet. Puis l'opérateur libère l'objet 2.

La libération de l'objet peut s'effectuer par déplacement d'au moins une partie des crochets 13, 14, 15 par application d'un effort dans un sens opposé à l'effort de rappel auquel ils sont soumis du fait de la flexibilité des bras 110, 120.

Préférentiellement, l'outil 9 est couplé à l'implant avant sa libération, pour bénéficier du maintien de l'implant 2 dans le dispositif 1, pour ne pas avoir à toucher manuellement l'implant. L'outil 9 peut alors être utilisé pour sortir l'implant par exemple par déplacement axial selon A2.

En variante, si selon un contexte d'utilisation donné, le fait de toucher manuellement l'implant n'est pas gênant, l'opérateur peut alors simplement libérer l'implant par déplacement d'un ou des systèmes de crochets, le saisir manuellement. On peut encore prévoir que l'opérateur puisse laisser glisser l'implant libéré hors du dispositif.

La libération de l'objet peut aussi s'effectuer par pivotement de l'objet 2 autour d'un axe APIV2 (virtuel) transversal à l'axe, dit axe de passage A1 selon lequel s'étend l'objet 2.

Le pivotement peut être réalisé manuellement ou à l'aide d'un outil 9.

Une référence dans toute la spécification à « un mode de réalisation » signifie qu'une fonctionnalité, une structure, ou une caractéristique particulière décrite en relation avec un mode de réalisation est incluse dans au moins un mode de réalisation de la présente invention. Ainsi, l'apparition de l'expression « dans un mode de réalisation » à divers emplacements dans toute la spécification ne fait pas nécessairement référence au même mode de réalisation. En outre, les fonctionnalités, les structures, ou les caractéristiques particulières peuvent être combinées de n'importe quelle manière appropriée dans un ou plusieurs modes de réalisation.

L'invention n'est pas limitée aux modes de réalisation illustrés dans les dessins.

De plus, le terme « comprenant » n'exclut pas d'autres éléments ou étapes. En outre, des caractéristiques ou étapes qui ont été décrites en référence à l'un des modes de réalisation exposés ci-dessus peuvent également être utilisées en combinaison avec d'autres caractéristiques ou étapes d'autres modes de réalisation exposés ci-dessus.

## Revendications

1. Dispositif pour le maintien et la libération d'un objet (2), tel qu'un implant médical,
**caractérisé en ce que** le dispositif comprend deux systèmes (11, 12) de crochet avec lesquels ledit objet (2) est apte à être couplé pour permettre auxdits systèmes (11, 12) de crochet de maintenir ensemble ledit objet (2),
lesdits systèmes (11, 12) de crochet étant configurés de telle sorte que, à l'état couplé dudit objet (2) auxdits systèmes (11, 12) de crochet, au moins l'un des systèmes (11, 12) de crochet est soumis à une force (F11, F12) de rappel permettant de maintenir l'objet couplé (2) auxdits systèmes (11, 12 ) de crochet, **caractérisé en ce que**, chaque système (11, 12) de crochet présentant au moins un crochet (13, 14) présentant un passage à travers lequel au moins une partie (22) dudit objet (2) est apte à s'étendre, ledit passage dudit au moins un crochet de chaque système de crochet (11, 12) présentant un axe (A1) de passage le long duquel au moins une partie (22) dudit objet (2) est apte à s'étendre, ledit au moins un crochet de chaque système de crochet présente une zone d'ouverture de crochet (131, 141, 151) configurée de manière à permettre une libération dudit objet (2) à travers ladite zone d'ouverture de crochet (131, 141, 151), par pivotement dudit objet (2) autour d'un axe (APIV2) orthogonal à l'axe (A1) de passage.

2. Dispositif selon la revendication 1, **caractérisé en ce que**, à l'état couplé dudit objet (2) auxdits systèmes (11, 12) de crochet, chacun desdits systèmes (11, 12) de crochet étant soumis à une force (F11, F12) de rappel,
lesdits systèmes (11, 12) de crochet sont configurés de telle sorte que la force de rappel auquel est soumis l'un desdits systèmes (11, 12) de crochet, tend à le déplacer en sens opposé au sens de la force (F12, F11) de rappel auquel est soumis l'autre système (12, 11) de crochet, l'objet étant maintenu couplé auxdits systèmes de crochet sous l'effet des forces (F11, F12) de rappel exercées en sens opposé par lesdits systèmes (11, 12) de crochet sur l'objet (2).

3. Dispositif selon la revendication 1 ou 2, **caractérisé en ce que** au moins l'un, de préférence chacun, des systèmes (11, 12) de crochet est configuré pour pouvoir être déplacé par rapport à l'autre système de crochet (12, 11) dans une position, dite position de libération, permettant de libérer l'objet par rapport aux systèmes (11, 12) de crochet.

4. Dispositif selon l'une quelconque des revendications précédentes, **caractérisé en ce qu'**au moins l'un, de préférence chacun, des systèmes (11, 12) de crochet comprend un bras (110, 120) qui est muni d'au moins un crochet (13, 14),
et **en ce que** ledit ou chaque bras (110, 120) est déplaçable, de préférence par flexion ou déformation élastique, pour permettre au système (11, 12) de crochet correspondant, d'être déplacé par rapport à l'autre système (12, 11) de crochet.

5. Dispositif selon l'une quelconque des revendications précédentes, **caractérisé en ce que** le dispositif comprend un corps (10) auquel est couplé chaque système (11, 12) de crochet.

6. Dispositif selon l'une quelconque des revendications précédentes, **caractérisé en ce que**, ledit passage dudit au moins un crochet de chaque système de crochet (11, 12) présentant un axe (A1) de passage le long duquel au moins une partie (22) dudit objet (2) est apte à s'étendre, les crochets sont, à l'état couplé de l'objet (2) auxdits systèmes de crochets, répartis à différentes positions le long dudit axe (A1) de passage.

7. Dispositif selon l'une quelconque des revendications précédentes, **caractérisé en ce que**, ledit passage dudit au moins un crochet de chaque système de crochet (11, 12) présentant un axe (A1) de passage le long duquel au moins une partie (22) dudit objet (2) est apte à s'étendre, ledit ou chaque système de crochet (11, 12) est déplaçable par flexion autour d'un axe orthogonal audit axe (A1) de passage.

8. Dispositif selon l'une quelconque des revendication précédentes, **caractérisé en ce que** l'un (12) des systèmes de crochet (11, 12) comprend un crochet (15) supplémentaire, à travers lequel au moins une partie (22) dudit objet (2) est apte à s'étendre,
ledit au moins un crochet (13) de l'autre système (11) de crochet étant apte à s'étendre, à l'état couplé de l'objet (2) aux systèmes (11, 12) de crochet, entre les deux crochets (14, 15) dudit système (12) de crochet.

9. Dispositif selon l'une quelconque des revendications précédentes, **caractérisé en ce que** au moins l'un (13) des crochets présente une portion d'extrémité (138) qui s'étend de manière à, lorsque l'objet (2) est couplé aux systèmes (11, 12) de crochet en étant soumis à la force (F11, F12) de rappel du ou de chacun des systèmes (11, 12) des crochets, s'opposer à une libération de l'objet (2) par déplacement à translation dans une direction contenue dans un plan orthogonal à l'axe (A1) selon lequel s'étend l'objet (2) à l'état maintenu de l'objet (2) par lesdits systèmes (11, 12) de crochet.

10. Dispositif selon l'une quelconque des revendications précédentes, **caractérisé en ce que** le dispositif comprend également un capot (190) monté coulissant entre une position, dite position de masquage, qui masque au moins partiellement les systèmes (11, 12) de crochet, et une autre position, dite position d'accès aux systèmes de crochet, qui permet à l'opérateur de déplacer les systèmes (11, 12) de crochet.

11. Dispositif selon la revendication 10, **caractérisé en ce que** le capot (190) est configuré de telle sorte que, en position de masquage, la longueur de l'objet, c'est-à-dire au moins ses extrémités opposées, reste visible.

12. Ensemble comprenant un dispositif conforme à l'une quelconque des revendications précédentes, et un objet (2) maintenu par lesdits systèmes (11, 12) de crochet.

13. Ensemble selon la revendication 12, **caractérisé en ce que** ledit objet (2), de préférence un implant médical, comprend un corps (22) de vis allongé et une tête (21) de vis, les systèmes (11, 12) de crochet venant en prise avec le corps (22) de vis allongé.

14. Ensemble selon la revendication 13, **caractérisé en ce que** l'extrémité du corps (22) de vis est contenue dans un logement (100) ménagé dans un corps (10) du dispositif.

15. Procédé de libération d'un objet couplé à un ensemble conforme à l'une quelconque des revendications 12 à 14, ledit procédé comprenant les étapes suivantes :
- lorsque qu'un capot (190) est présent, déplacement du capot, pour permettre l'accès aux systèmes (11, 12) de crochet et/ou découvrir une partie de l'objet précédemment masquée par le capot ;
- libération de l'objet (2) par :
i) déplacement d'au moins l'un, de préférence de chacun, des systèmes (11, 12) de crochet en appliquant un effort sur au moins l'un, de préférence chacun, des systèmes (11, 12) de crochet, dans un sens opposé à l'effort de rappel dudit système (11, 12) de crochet,
ou
ii) pivotement de l'objet (2) autour d'un axe (APIV2) transversal à l'axe, dit axe de passage selon lequel s'étend l'objet (2), ledit pivotement étant effectué de préférence à l'aide d'un outil (9) couplé à l'objet (2).

## Patentansprüche

1. Vorrichtung zum Halten und Lösen eines Gegenstands (2) wie z.B. eines medizinischen Implantates,
**dadurch gekennzeichnet, dass** die Vorrichtung zwei Hakensysteme (11, 12) umfasst, mit denen die Vorrichtung (2) gekoppelt werden kann, damit die Hakensysteme (11, 12) den Gegenstand (2) zusammenhalten können,
wobei die Hakensysteme (11, 12) derart konfiguriert sind, dass, wenn der Gegenstand (2) mit den Hakensystemen (11, 12) gekoppelt ist, mindestens eines der Hakensysteme (11, 12) einer Rückstellkraft (F11, F12) unterworfen ist, die die Aufrechterhaltung der Kopplung des Gegenstandes (2) mit den Hakensystemen (11, 12) ermöglicht, **dadurch gekennzeichnet, dass**, da jedes Hakensystem (11, 12) mindestens einen Haken (13, 14) mit einem Durchgang, durch den sich mindestens ein Teil (22) des Gegenstandes (2) erstrecken kann, aufweist, wobei der Durchgang des mindestens einen Hakens jedes Hakensystems (11, 12) eine Durchgangsachse (A1) aufweist, längs derer sich mindestens ein Teil (22) des Gegenstandes (2) erstrecken kann, wobei der mindestens ein Haken jedes Hakensystems einen Hakenöffnungsbereich (131, 141, 151) aufweist, der derart konfiguriert ist, dass ein Lösen des Gegenstandes (2) über den Öffnungsbereich (131, 141, 151) durch Schwenken des Gegenstandes (2) um eine orthogonal zur Durchgangsachse (A1) verlaufenden Achse (APIV2) möglich ist.

2. Vorrichtung nach Anspruch 1, **dadurch gekennzeichnet, dass**, wenn der Gegenstand (2) mit den Hakensystemen (11, 12) gekoppelt ist und jedes der Hakensysteme (11, 12) einer Rückstellkraft (F11, F12) unterworfen ist,
die Hakensysteme (11, 12) derart konfiguriert sind, dass die Rückstellkraft, der eines der Hakensysteme (11, 12) unterworfen ist, dazu neigt, dieses gegen die Richtung der Rückstellkraft (F12, F11), der das andere Hakensystem (12, 11) unterworfen ist, zu verschieben, wobei die Kopplung des Gegenstandes mit den Hakensystemen unter den in entgegengesetzter Richtung durch die Hakensysteme (11, 12) auf den Gegenstand (2) ausgeübten Rückstellkräften (F12, F11) aufrechterhalten wird.

3. Vorrichtung nach Anspruch 1 oder 2, **dadurch gekennzeichnet, dass** mindestens eines, vorzugsweise jedes, der Hakensysteme (11, 12) derart konfiguriert ist, dass es in Bezug auf das andere Hakensystem (12, 11) in eine Stellung ("Lösestellung") gebracht zu werden, die die Lösung des Gegenstandes in Bezug auf die Hakensysteme (11, 12) ermöglicht.

4. Vorrichtung nach einem der vorstehenden Ansprüche, **dadurch gekennzeichnet, dass** mindestens eines, vorzugsweise jedes, der Hakensysteme (11, 12) einen Arm (110, 120) umfasst, der mit mindestens einem Haken (13, 14) versehen ist,
und das der bzw. jeder Arm (110, 120), vorzugsweise durch Beugung oder elastische Verformung, verschiebbar ist, um zu ermöglichen, dass das entsprechende Hakensystem (11, 12) in Bezug auf das andere Hakensystem (12, 11) verschoben wird.

5. Vorrichtung nach einem der vorstehenden Ansprüchhe,
**dadurch gekennzeichnet, dass** die Vorrichtung ein Hauptteil (10) umfasst, mit dem jedes Hakensystem (11, 12) gekoppelt ist.

6. Vorrichtung nach einem der vorstehenden Ansprüche,
**Dadurch gekennzeichnet, dass**, da der Durchgang des mindestens einen Hakens jedes Hakensystems (11, 12) eine Durchgangsachse (A1) aufweist, längs derer sich mindestens ein Teil (22) des Gegenstandes (2) erstrecken kann, die Haken in unterschiedlichen Positionen längs der Durchgangsachse (A1) verteilt sind, wenn der Gegenstand (2) mit den Hakensystemen gekoppelt ist.

7. Vorrichtung nach einem der vorstehenden Ansprüche,
**dadurch gekennzeichnet, dass**, da der Durchgang des mindestens einen Hakens jedes Hakensystems (11, 12) eine Durchgangsachse (A1) aufweist, längs derer sich mindestens ein Teil (22) des Gegenstandes (2) erstrecken kann, das bzw. jedes Hakensystem (11, 12) durch Beugung um eine orthogonal zur Durchgangsachse (A1) verlaufende Achse verschiebbar ist.

8. Vorrichtung nach einem der vorstehenden Ansprüche, **dadurch gekennzeichnet, dass** eines (12) der Hakensysteme (11, 12) einen zusätzlichen Haken (15) umfasst, durch den sich mindestens ein Teil (22) des Gegenstandes (2) erstrecken kann,
wobei der mindestens eine Haken (13) des anderen Hakensystems (11) sich zwischen den beiden Haken (14, 15) des Hakensystems (12) erstrecken kann, wenn der Gegenstand (2) mit den Hakensystemen (11, 12) gekoppelt ist.

9. Vorrichtung nach einem der vorhergehenden Ansprüche, **dadurch gekennzeichnet, dass** mindestens einer (13) der Haken einen Endabschnitt (138) aufweist, der sich so erstreckt, dass er, wenn der Gegenstand mit den Hakensystemen (11, 12) gekoppelt ist, indem er der Rückstellkraft (F11, F12) der Rückstellkraft des oder jedes der Hakensysteme (11, 12) ausgesetzt ist, einer Freigabe des Gegenstandes (2) durch Translationsbewegung in einer Richtung entgegenwirkt, die in einer Ebene enthalten ist, die orthogonal zu der Achse (A1) ist, entlang der sich der Gegenstand (2) in dem Zustand erstreckt, in dem der Gegenstand (2) von den Hakensystemen (11, 12) gehalten wird.

10. Vorrichtung nach einem der vorstehenden Ansprüche, **dadurch gekennzeichnet, dass** die Vorrichtung auch eine Abdeckung (190) umfasst, die zwischen einer Stellung ("Maskierstellung"), die die Hakensysteme (11, 12) mindestens zum Teil maskiert, und einer anderen Stellung ("Zugriffsstellung"), die dem Bediener die Verschiebung der Hakensysteme (11, 12) ermöglicht, verschiebbar ist.

11. Vorrichtung nach Anspruch 10, **dadurch gekennzeichnet, dass** die Abdeckung (190) derart konfiguriert ist, dass die Länge des Gegenstandes, d.h. mindestens dessen entgegengesetzte Enden, in der Maskierstellung sichtbar bleibt.

12. Einrichtung, umfassend eine Vorrichtung nach einem der vorstehenden Ansprüche und einen von den Hakensystemen (11, 12) gehaltenen Gegenstand (2).

13. Einrichtung nach Anspruch 12, **dadurch gekennzeichnet, dass** der Gegenstand (2), vorzugsweise ein medizinisches Implantat, einen länglichen Schraubkörper (22) und einen Schraubkopf (21) umfasst, wobei die Hakensysteme (11, 12) in den länglichen Schraubkörper (22) eingreifen.

14. Einrichtung nach Anspruch 13, **dadurch gekennzeichnet, dass** das Ende des Schraubkörpers (22) in einem in einem Hauptteil (10) der Vorrichtung untergebrachten Gehäuse (100) enthalten ist.

15. Verfahren zum Lösen eines mit einer Einrichtung nach einem der Ansprüche 12 - 14 gekoppelten Gegenstandes, wobei das Verfahren die Schritte umfasst:
- bei Vorhandensein einer Abdeckung (190): Verschieben der Abdeckung, um den Zugriff auf die Hakensysteme (11, 12) zu gewähren und/oder einen vorher durch die Abdeckung maskierten Teil des Gegenstandes aufzudecken;
- Lösen des Gegenstandes (2) durch:
i) Verschieben mindestens eines, vorzugsweise jedes, der Hakensysteme (11, 12) durch Anwenden einer Kraft auf mindestens eines, vorzugsweise jedes, der Hakensysteme (11, 12) in eine der Rückstellkraft der Hakensysteme (11, 12) entgegengesetzte richtung,
oder
ii) Schwenken des Gegenstandes (2) um eine quer zur Achse ("Durchgangsachse"), längs derer sich der Gegenstand (2) erstreckt, verlaufende Achse (APIV2), wobei die Schwenkung vorzugsweise mithilfe eines mit dem Gegenstand (2) gekoppelten Werkzeugs (9) vollzogen wird.

## Claims

1. A device for holding and releasing an object (2), such as a medical implant,
**characterized in that** the device comprises two hook systems (11, 12) to which said object (2) is able to be coupled so as to allow said hook systems (11, 12) to jointly hold said object (2),
said hook systems (11, 12) being configured such that, in the state in which said object (2) is coupled to said hook systems (11, 12), at least one of the hook systems (11, 12) is subjected to a return force (F11, F12) that makes it possible to keep the object (2) coupled to said hook systems (11, 12),
**characterized in that**, each hook system (11, 12) having at least one hook (13, 14) that has a passage through which at least a part (22) of said object (2) is able to extend, said passage of said at least one hook of each hook system (11, 12) having a passage axis (A1) along which at least a part (22) of said object (2) is able to extend, said at least one hook of each hook system has a hook opening zone (131, 141, 151) configured so as to allow said object (2) to be released through said hook opening zone (131, 141, 151), by pivoting of said object (2) about an axis (APIV2) orthogonal to the passage axis (A1).

2. The device according to claim 1, **characterized in that**, in the state in which said object (2) is coupled to said hook systems (11, 12), each of said hook systems (11, 12) being subjected to a return force (F11, F12),
said hook systems (11, 12) are configured such that the return force to which one of said hook systems (11, 12) is subjected tends to move it in the direction opposite the direction of the return force (F12, F11) to which the other hook system (12, 11) is subjected, the object being kept coupled to said hook systems under the effect of the return forces (F11, F12) applied in opposite directions to the object (2) by said hook systems (11, 12).

3. The device according to claim 1 or 2, **characterized in that** at least one, preferably each, of the hook systems (11, 12) is configured to be able to be moved with respect to the other hook system (12, 11) into a position, referred to as the release position, that makes it possible to release the object from the hook systems (11, 12).

4. The device according to any one of the preceding claims, **characterized in that** at least one, preferably each, of the hook systems (11, 12) comprises an arm (110, 120) that is provided with at least one hook (13, 14),
and **in that** said or each arm (110, 120) is movable, preferably by bending or elastic deformation, so as to allow the corresponding hook system (11, 12) to be moved with respect to the other hook system (12, 11).

5. The device according to any one of the preceding claims, **characterized in that** the device comprises a body (10) to which each hook system (11, 12) is coupled.

6. The device according to any one of the preceding claims, **characterized in that**, said passage of said at least one hook of each hook system (11, 12) having a passage axis (A1) along which at least a part (22) of said object (2) is able to extend, the hooks are distributed, in the state in which the object (2) is coupled to said hook systems, at different positions along said passage axis (A1).

7. The device according to any one of the preceding claims, **characterized in that**, said passage of said at least one hook of each hook system (11, 12) having a passage axis (A1) along which at least a part (22) of said object (2) is able to extend, said or each hook system (11, 12) is movable by bending about an axis orthogonal to said passage axis (A1).

8. The device according to any one of the preceding claims, **characterized in that** one (12) of the hook systems (11, 12) comprises an additional hook (15), through which at least a part (22) of said object (2) is able to extend,
said at least one hook (13) of the other hook system (11) being able to extend, in the state in which the object (2) is coupled to the hook systems (11, 12), between the two hooks (14, 15) of said hook system (12).

9. The device according to any one of the preceding claims, **characterized in that** at least one (13) of the hooks has an end portion (138) that extends in such a way that, when the object (2) is coupled to the hook systems (11, 12) by being subjected to the return force (F11, F12) of the or each of the hook system(s) (11, 12), said end portion opposes a release of the object (2) by translational movement in a direction contained in a plane orthogonal to the axis (A1) along which the object (2) extends in the state in which the object (2) is held by said hook systems (11, 12).

10. The device according to any one of the preceding claims, **characterized in that** the device also comprises a cover (190) mounted so as to slide between a position, referred to as the concealing position, which at least partially conceals the hook systems (11, 12), and another position, referred to as the position for accessing the hook systems, which allows the operator to move the hook systems (11, 12).

11. The device according to claim 10, **characterized in that** the cover (190) is configured such that, in the concealing position, the length of the object, that is to say, at least its opposite ends, remains visible.

12. An assembly comprising a device according to any one of the preceding claims, and an object (2) held by said hook systems (11, 12).

13. The assembly according to claim 12, **characterized in that** said object (2), preferably a medical implant, comprises an elongate screw body (22) and a screw head (21), the hook systems (11, 12) engaging with the elongate screw body (22).

14. The assembly according to claim 13, **characterized in that** the end of the screw body (22) is contained in a housing (100) provided in a body (10) of the device.

15. A method for releasing an object coupled to an assembly according to any one of claims 12 to 14, said method comprising the following steps:
- when a cover (190) is present, moving the cover so as to allow access to the hook systems (11, 12) and/or to uncover a part of the object previously concealed by the cover;
- releasing the object (2) by:
i) moving at least one, preferably each, of the hook systems (11, 12) by applying a force to at least one, preferably each, of the hook systems (11, 12) in a direction opposite to the return force of said hook system (11, 12),
or
ii) pivoting the object (2) about an axis (APIV2) transverse to the axis, referred to as the passage axis, along which the object (2) extends, said pivoting being brought about preferably with the aid of a tool (9) coupled to the object (2).
